# EUROPÄISCHE PATENTSCHRIFT

(11) **EP 0 889 723 B1**
(45) Veröffentlichungstag und Bekanntmachung des Hinweises auf die Patenterteilung: **05.06.2002**
(21) Anmeldenummer: 97914228.8
(22) Anmeldetag: 12.03.1997
(51) Int. Cl.: A61K 9/70, A61M 35/00, A61M 37/00

(54) **TRANSDERMALES THERAPEUTISCHES SYSTEM MIT GERINGER APPLIKATIONSDICKE UND HOHER FLEXIBILITÄT SOWIE HERSTELLUNGSVERFAHREN**
TRANSDERMAL THERAPEUTIC SYSTEM WITH SMALL APPLICATION-AREA THICKNESS AND GREAT FLEXIBILITY, AND PRODUCTION PROCESS
SYSTEME THERAPEUTIQUE TRANSDERMIQUE A FAIBLE EPAISSEUR DE LA ZONE D'APPLICATION ET A GRANDE SOUPLESSE, ET SON PROCEDE DE FABRICATION

(30) Priorität: 25.03.1996 DE 19611684; 04.03.1997 DE 19708674
(43) Veröffentlichungstag der Anmeldung: 13.01.1999
(73) Patentinhaber: LTS LOHMANN Therapie-Systeme AG, 56626 Andernach (DE)
(72) Erfinder: VON KLEINSORGEN, Reinhard, D-56170 Bendorf (DE); VON KLEINSORGEN, Britta, D-56170 Bendorf (DE)
(74) Vertreter: Flaccus, Rolf-Dieter, Dr.
(86) Internationale Anmeldenummer: EP9701252
(87) Internationale Veröffentlichungsnummer: WO9735564

(56) Entgegenhaltungen:
- EP-A- 0 566 816

## Beschreibung

Die Erfindung betrifft ein Schichtsystem zur Applikation einer wirkstoffabgebenden Filmschicht auf einem Substrat, insbesondere auf der Haut, zwecks kontrollierter Abgabe von Wirkstoffen an menschliche oder tierische Haut, wobei sich das System durch geringe Applikationsdicke und hohe Flexibilität auszeichnet; sowie dafür geeignete Herstellungsverfahren.

Systeme zur kontrollierten Abgabe von Wirkstoffen an menschliche oder tierische Haut sind unter anderen als sogenannte Transdermale Therapeutische Systeme (TTS) oder "Transdermal Delivery Systems" (TDS) bekannt.
Bei diesen Systemen wird nach Aufbau und Art der Wirkstofffreisetzung zwischen sogenannten Beutel- oder Reservoir-Systemen und Matrix-Systemen unterschieden. Im ersten Fall bestehen solche Systeme aus einem Wirkstoff beinhaltenden flachen Beutel dessen eine Seite für Wirk- und Hilfsstoffe undurchlässig und dessen gegenüberliegende Seite als Steuermembran semipermeabel ausgebildet und zwecks Haftung auf der Haut mit Kleber beschichtet ist. Infolge seines komplizierten Aufbaus ist die Herstellung des Systems sehr aufwendig, da die Einzelkomponenten separat hergestellt und dann zu einem System zusammengefügt werden müssen.
Weiterhin sind aufgrund der Dicke des Systems die Trageigenschaften beeinträchtigt. Außerdem besteht bei Beutel-Systemen die Gefahr des sogenannten "Drug Dumping", d.h. der plötzlichen weitgehenden Freisetzung des Wirkstoffes an die Haut, z.B. infolge physikalischer Zerstörung der Membran bzw. des Beutels. Die EP 0 285 563 beschreibt z.B. ein transdermales therapeutisches System dieser Art für die kombinierte Applikation von Oestrogenen und Gestagenen.
Aus der US-PS 4 624 665 sind Systeme bekannt, die im Reservoir den Wirkstoff in mikroverkapselter Form enthalten. Das Reservoir ist eingebettet zwischen Rückschicht und Membran. Der Rand des Systems ist mit einem Haftkleber ausgerüstet.
Aufbau und die Herstellung dieses Systems sind sehr kompliziert, da der Wirkstoff mikroverkapselt und homogen verteilt werden muß, um dann zwischen Rückschicht und Membran eingebettet zu werden. Außerdem muß das System mit einem klebenden Rand versehen und mit einer Schutzschicht abgedeckt werden.
Matrix-Systeme bestehen üblicherweise aus einer für Wirkund Hilfsstoff undurchlässigen, der Haut abgewandten Rückschicht und einer Kleberschicht, in der Wirkstoff verteilt ist. Zum Schutz der Kleberschicht ist diese mit einer abhäsiv ausgestatteten Schutzfolie versehen, die vor der Applikation entfernt werden muß. Ein solches System ist in der DE-OS 20 06 969 beschrieben, bei dem empfängnisverhütende Substanzen in die Klebstoffkomponente oder den Klebstoffilm eingearbeitet sind. Aus dieser Schrift ist zu entnehmen, daß der Klebstoffilm ein Acrylat sein kann.
Matrix-Systeme weisen allgemein den Nachteil auf, daß sie ähnlich wie Etiketten ausgehend von einem selbstklebenden Laminat auf einer abhäsiv ausgerüsteten Schutzschicht ausgestanzt werden. Die Stanzlinge auf der Schutzschicht werden in einem weiteren Schritt vereinzelt, um so das fertige TTS darzustellen. Die Bereiche zwischen den einzelnen Stanzlingen, bestehend aus wirkstoffhaltiger Kleberschicht und Rückschicht, sind als wirkstoffhaltiger Abfall zu entsorgen. Lösungen zur Vermeidung dieses Abfalls sind beispielsweise aus der DE 39 39 376, und der DE 29 08 432 sowie aus der EP 0 400 078 B1 bekannt.

Nachteilig ist bei bekannten Matrix-Systemen eine durch das Herstellverfahren und die Art der Applikation bedingte Dicke des Systems, welche die Flexibilität des Systems und damit dessen Trageigenschaften in unerwünschter Weise beeinträchtigt, wogegen der Tragecomfort des Systems mit abnehmender Dicke zunimmt.

Grundsätzlich ist bekannt, daß auch dünne Filme zur transdermalen Verabreichung von Wirkstoffen verwendet werden können; allerdings ist die Handhabung solcher Filme, insbesondere bei der Applikation, aufgrund der geringen Dicke problematisch. In EP-A 0 566 816 wurde eine Darreichungsform mit einem wirkstoffhaltigen Film beschrieben, der sich zwischen einer Trägerschicht und einer Schutzschicht befindet. Damit der klebend mit der Trägerschicht verbundene Film bei der Applikation von der Trägerschicht entfernt werden kann, befindet sich zwischen dieser Schicht und der Filmschicht ein Abnahme- oder Abziehmittel. Dabei handelt es sich um ein Folienmaterial oder ein Laminat, das mit Teflon oder einem anderen abhäsiven Material beschichtet ist, um auf diese Weise bei der Applikation die Ablösung des Trägers von der klebenden Seite des Films zu ermöglichen. Bei der Herstellung wird die genannte Abnahme- oder Abziehfolie in einem zusätzlichen Arbeitsschritt auf die wirkstoffhaltigen Filmstücke, die vorher auf die Schutzschicht aufgebracht wurden, auflaminiert. Die Filmstücke werden durch Zuschneiden aus dem bahnförmigen Filmmaterial hergestellt.

Weiterhin weisen bekannte Matrix-Systeme insofern technische Grenzen auf, als es mit ihnen nicht gelingt, gleichzeitig oder in zeitlicher Folge alternative Dosierungen, Wirkstoffkonzentrationen oder unterschiedliche chemische Zusammensetzungen des Systems anzubieten, die sich durch den Wirkstoff oder eine spezielle Wirkstoffkombination voneinander unterscheiden und eine gewünschte zeitlich oder örtlich unterschiedliche Applikation mehrerer Wirkstoffe ermöglichen.

Der vorliegenden Erfindung liegt die Aufgabe zugrunde, ein Schichtsystem zur Applikation einer den Wirkstoff abgebenden Filmschicht auf einem Substrat anzugeben, welches die vorgenannten Nachteile und Schwierigkeiten vermeidet und aufgrund verminderter Dicke sehr gute Flexibilität und damit verbesserte Trageigenschaften aufweist, und das eine verbesserte Handhabung bei der Applikation aufweist und durch Druckverfahren herstellbar ist. Dabei soll die Herstellung von sogenannten "Multi-Dose"-Einheiten ermöglicht werden, die aus mehreren abteilbaren "Single-Dose"-Systemen bestehen. Diese können sich hinsichtlich der Parameter Parameter Wirkstoffart/Wirkstoff konzentraton/ Systemfläche/Systemdicke/ chemische Zusammensetzung des Systems unterscheiden und sind damit in der Lage, mittels einer "Multi-Dose"-Einheit gleichzeitig oder in zeitlicher Folge alternative Dosierungen anzubieten.

Die Lösung der Aufgabe wird bei einem Schichtsystem zur Applikation einer Wirkstoff abgebenden Filmschicht auf einem Substrat mit der Erfindung entsprechend den kennzeichnenden Merkmalen von Anspruch 1 erreicht.

Durch die Konsistenz-Änderung der Trennschicht, z. B. durch Auflösung oder Verflüssigung, kann auf einfache Weise die Ablösung der Trägerschicht bei der Applikation erreicht werden. Vorteilhaft ist ferner, daß diese Trennschichten durch Druckverfahren herstellbar sind.

Damit wird die vorliegende Erfindung den Ansprüchen der Aufgabenstellung gerecht, indem sie ein System zur Verfügung stellt, welches aufgrund seiner geringen Dicke infolge guter Flexibilität verbesserte Trageigenschaften aufweist. Außerdem gestattet die dem erfindungsgemäßen System zugrundeliegende Herstelltechnologie die Herstellung von sogenannten "Multi-Dose"-Einheiten, die aus abteilbaren "Single-Dose"-Systemen bestehen. Diese "Single-Dose"-Systeme einer "Multi-Dose"-Einheit können gleich oder unterschiedlich und die Unterschiede verursachenden Parameter können
- Art des Wirkstoffs,
- Konzentration des Wirkstoffs pro System,
- Fläche eines Systems,
- Dicke eines Systems und
- chemische Zusammensetzung des Systems sein.

Damit wird ermöglicht, mittels einer "Multi-Dose"-Abgabeeinheit gleichzeitig oder in zeitlicher Folge alternative Dosierungen anzubieten. Als "Multi-Dose"-Einheiten entsprechend der vorliegenden Erfindung können auch "Single-Dose"-Systeme vorgesehen werden, die sich durch den Wirkstoff selbst und durch Wirkstoffkombinationen voneinander unterscheiden, wodurch eine bestimmte gewünschte zeitlich unterschiedliche Applikation mehrerer Wirkstoffe ermöglicht wird.

Damit bietet das erfindungsgemäße System eine kostengünstigere Alternative zu den bisherigen Systemen an.

Überraschenderweise hat sich gezeigt, daß sich das Prinzip der sogenannten "Decalcomanias" = "Decals" bzw. "Tattoos" als Wirkstoffträger bzw. Applikationssysteme zur transdermalen Therapie besonders gut eignen. Der Begriff "Tattoo", ehedem in Verbindung mit "künstlerischer" Pigmentierung der Haut verwendet, hat sich in der Literatur auch für den Einsatz von "Decalcomanias" zu Dekorationszwecken durchgesetzt.
"Decals" sind z.B. aus der Keramikindustrie bekannte "Abzieh-" oder "Abschiebebilder" für Dekorierungszwecke. Sie bestehen aus einem mit einer lösemittellöslichen Trennschicht versehenen Papierträger, auf dem Lackschichten aufgebracht sind. Vor bzw. während des Zusammenführens der Lackschicht mit dem Steingut wird das Papier mit Lösemittel angefeuchtet, wobei die Trennschicht in Lösung geht. Die Lackschicht kann jetzt vom Papierträger abgeschoben werden, um ihren Platz auf dem zu dekorierenden Substrat zu finden. Der Verbund kann durch Einbrennen verstärkt werden.

Ein Decal zur transdermalen Anwendung von Wirkstoffen hat vorteilhaft folgenden Aufbau:

Auf einer Trägerschicht, bestehend entweder aus einem Papier, einem Gewebe, einem Vlies oder einer für Lösungsmittel durchlässigen Polymerschicht, z.B. einer Polymermembran, ist eine lösliche Trennschicht aufgebracht. Die Differenzierung dieses Laminats in zwei Einzelschichten kann durch eine Sperrsicht unterstützt werden, die verhindert, daß beim Auftragen der Trennschicht Bestandteile in die Trägerschicht gelangen.
Wesentlich ist eine kapillaraktive unlösliche Trägerschicht, die mit einer löslichen Trennschicht versehen ist. Die Dicke der Trägerschicht liegt im Bereich von 20 - 200 µm, vorzugsweise 50-120 µm und in einer bevorzugten Form bei 60 - 90 µm. Die Trennschicht weist eine Dicke von 5 - 50 µm, vorzugsweise 20 - 40 µm auf. Auf die mit der Trägerschicht verbundenen Trennschicht ist eine wirkstoffhaltige Filmschicht aufgebracht. Diese kann ein Laminat sein, dessen Einzelschichten unterschiedlich sein können. So kann z.B. die der Trennschicht zugewandte Schicht des Laminats wirkstofffrei sein. Um einen besseren Verbund mit der Haut zu erzielen, kann die der Trennschicht abgewandte Schicht des Laminats klebend ausgestattet sein. Die einzelnen Schichten des Laminates können hinsichtlich ihres Polymergrundkörpers gleich oder unterschiedlich sein. Bei einer "Multi-Dose"-Einheit ist die mit der Trennschicht versehene Trägerschicht mit mehreren flächenförmigen inselartigen Bereichen der wirkstoffhaltigen Filmschicht bedruckt. Als Druckverfahren eignen sich alle dem Fachmann bekannte Druckverfahren bzw. Sprühverfahren oder Düsenauftragsverfahren, die es gestatten, eine wirkstoffhaltige Filmschicht mit der geforderten Gewichtskonstanz aufzutragen.
Die wirkstoffhaltige Filmschicht kann dabei in einem oder mehreren Einzeldruckschritten aufgetragen werden, wobei ähnlich wie bei einem Mehrfarbendruck auch Teilbereiche des Vordruckes bedruckt werden können.
Bei der erfindungsgemäßen Ausführungsform hat es sich als vorteilhaft erwiesen, die Filmschicht durch Siebdruck aufzutragen. Hierdurch ist es möglich, auf einem Trägerabschnitt mehrere in ihrer Größe unterschiedliche inselartige wirkstoffhaltige Filmschichtbereicbe aufzutragen, die sich ähnlich wie bei einem "Mehrfarbendruck" in Ihrer Zusammensetzung, Schichtstärke und Wirkstoff (unterschiedliches Pigment) unterscheiden.

Aufgrund der äußerst geringen Dicke und optimaler Flexibilität ist das erfindungsgemäße System im Gegensatz zu herkömmlichen Systemen geeignet, auch auf schwierige Stellen des Körpers dauerhaft appliziert zu werden, z.B. am Bereich des Ohres, am Genitalbereich oder an Fuß- und Fingernägeln. Die Dekormöglichkeiten erlauben ebenfalls ein Tragen auf Körperoberflächen, die von Kleidung nicht abgedeckt sind.

In einer besonderen Ausführungsform, in der die Filmschicht keine zusätzliche Haftkleberschicht trägt, weist ein Schichtbereich der Filmschicht auf der der Trennschicht gegenüberliegenden Seite zum Zeitpunkt der Applikation Eigenklebrigkeit auf. Dies wird dadurch erreicht, daß der Schichtbereich während der Auflösung der Trennschicht mit angefeuchtet wird und die angequollenen Bereiche der polymeren Filmschicht klebende Eigenschaften erhalten, die einen Verbund mit der Haut ermöglichen, jedoch auf der der Haut abgewandten Seite der Filmschicht nach Abdunsten des Lösungsmittels verloren gehen.
Die Dicke der Filmschicht liegt im Bereich von 5-50 µm, vorzugsweise 5-30 µm und in einer besonderen Ausführungsform 10-25 µm.
Die oben aufgeführte Filmschicht bzw. die einzelnen Filmschichtbereiche oder Filmschichtinseln flächenabdeckend mit einer Schutzschicht, die abhäsiv ausgestattet sein kann, abgedeckt. Bei inselartiger Ausführungsform der Filmschichtbereiche auf einer gemeinsamen Trägerschicht im "Multi-Dose"-System kann die Schutzschicht und die Trägerschicht im Bereich zwischen den "Inseln" perforiert sein, um Teilbereiche als "Single Dose" durch Abreißen aus dem Gesamtsystem entnehmen zu können. Die Dicke der Schutzschicht liegt im Bereich von 50 - 100 µm.

Zwecks Applikation wird die Schutzschicht des Systems entfernt und die Filmschicht mit Trägerschicht derart auf die Applikationsstelle aufgebracht, daß die Trägerschicht von der Applikationsstelle abgewandt ist, worauf die Benetzung mit Lösemittel erfolgt. In einer bevorzugten Ausführungsform wird als Lösemittel Wasser eingesetzt. Aufgrund der Kapillareigenschaften des Trägers gelangt das Wasser an die für diesen Fall wasserlöslich ausgebildete Trennschicht, die in ihrem an- beziehungsweise aufgelösten Zustand den Verbund zur Filmschicht verliert, so daß die Trägerschicht von der Filmschicht abgezogen werden kann und die Filmschicht auf der Applikationsstelle verbleibt.

Für den Fall, daß die Trennschicht wasserlöslich ausgestattet ist, stellt sie chemisch z.B. ein Saccharid bzw. ein Polysaccharid, ein Polyhydroxyalkohol, Polyvinylpyrrolidon oder ein anderes in Wasser lösliches Polymer wie Polyethylenglykol oder Gelatine dar.
Für den Fall, daß die Trennschicht fettlöslich ausgestattet ist, stellt sie chemisch ein Triglycerid oder ein Wachs dar. In einer besonderen Ausführungsform kann hier die Trennschicht derart ausgestattet werden, daß sie unter Wärmeeinwirkung flüssig wird und dadurch den Verbund zur Filmschicht verliert.
Die Filmschicht kann aus filmbildenden Polymeren bestehen. Es kommen in Betracht: Hydroxypropylcellulose, Hydroxypropylmethylcellulose, Polyvinylpyrrolidon, Vinylpyrrolidon-Vinylacetat-copolymerisat 60:40, Ethylcellulose, Acryl- und Methacrylsäureester-Copolymerisate mit Tritmethylammoniummethylacrylat, Copolymerisate von Dimethylaminomethacrylsäure und neutralen Methacrylsäureestern, Schellack, Celluloseacetatphtalat, Hydroxpropylmethylcellulosephtalat, Polymerisate von Methacrylsäure und Methacrylsäureestern, Acrylsäureäthylester-Methacrylsäuremethylester-Copolymersiat 70:30, Methacrylsäure-Acrylsäuremethylester-Copolymersat 50:50, Gelatine, Polyvinylacetat, Methacrylat, Acrylatdispersionen, Polyether-Polyamid-Block-copolymer, Polyethylen-Methyl-Methacrylat-Block-Copolymer, Polyurethane, Polyester-Block-Copolymere, Polyisobutylen-Styrol-Styrol-Copolymere, Styrol-Butadien-Styrol-Isopren-Copolymere, Ethylen-Vinylacetat-Copolymere, Polyamid, Nitrocellulose sowie dem Fachmann bekannte weitere Lack- bzw. Filmbildner. Der Zusatz von Weichmachern zu diesen Filmbildnern ergibt sich zwangsläufig entsprechend der geforderten notwendigen Flexibilität des Films.
Diese Filmschicht kann in Form eines Laminats durch unten aufgeführte Bestandteile in der der Applikationsstelle zugewandten Schicht haftklebend ausgeführt sein. Als Bestandteile in diesem Sinne eignen sich alle dem Fachmann bekannten handelsüblichen Kleber, die auch zur Wundversorgung in Form von Verbänden und Pflastern eingesetzt werden, wie z.B. Kleber auf der Basis von Acrylaten, Polyisobutylen, Polyurethanen, Silikonen etc. Die Schutzschicht bestehend aus einem mit einem Silikonharz beschichteten Papier oder Polymer, ist dem Fachmann in unterschiedlichen Ausführungsformen bekannt und im einschlägigen Handel als Kleberschutzschicht oder "Release-Liner" mit einer dem Kleber angepaßten Silikonisierung zu beziehen.
Die in das System einstanzbare Perforation stellt eine gesonderte Systemvariante dar, die gestattet, eine Einheit mit mehrerer gleichen oder unterschiedlichen Dosierungen und Wirkungszeiträumen zu erhalten. Dies ist insbesondere dann sinnvoll, wenn ein oder mehrere Arzneimittel oder Kombination über einen bestimmten Zeitraum in einer bestimmten Dosisstärke verabreicht werden sollen. So kann die Trägerschicht mittels Siebdruck mit gleichen oder unterschiedlichen Arzneimittel und oder Arzneimittelkombinationen in Form von voneinander beabstandeten Arealen bedruckt sein.
Gewünscht wird dies z.B. bei der cyclischen Hormonbehandlung mit Estradiol oder Gestagenen.

Unter der Bezeichnung "Wirkstoffe" im Zusammenhang mit der vorliegenden Erfindung werden chemische Elemente, organische und anorganische Verbindungen verstanden, die aus den sie enthaltenden Bestandteilen der gattungsgemäßen Vorrichtung herauswandern können und dadurch einen angestrebten Effekt hervorrufen. Unter den Anwendungsgebieten der erfindungsgemäßen Vorrichtung ist die Humanund Tiermedizin sowie die Anwendung an Pflanzen von besonderer Bedeutung.

Die abzugebenden Wirkstoffe dienen vorzugsweise der dermalen Behandlung lokaler Hauterkrankungen, der intraund transdermalen Behandlung von Erkrankungen, der Wundbehandlung oder der Pflege der Haut in kosmetischen Zubereitungen. In einer besonderen Ausführungsform dient die Filmschicht als Träger von leicht flüchtigen Wirkstoffen, die nach außen, d.h. in Richtung der der Haut abgewandten Seite abgegeben werden. Hier sind ebenfalls Geruchsstoffe wie Parfümöle einbezogen, die einen Wirkstoffcharakter im weitesten Sinne besitzen (olfaktorische Stoffe).

Zur dermalen Behandlung lokaler Hauterkrankungen werden Lokalanästhetika, Lokalantibiotika, Antiseptika, Antimykotika, Antihistaminika und juckreizstillende Arzneistoffe, Keratolytika und ätzende Arzneistoffe, Virustatika, Antiskabiesmittel, Steroide, sowie verschiedene Substanzen zur Behandlung von Akne, Psoriasis, Lichtdermatosen oder Präcancerosen verwendet. Zu den Wirkstoffen, die intradermal applizierbar sind gehören z.B. steroidale und nichtsteroidale Antirheumatika, Lokalanästhetika, durchblutungsfördernde Substanzen oder Vasoprotektoren und -konstriktoren zur Behandlung von Gefäßerkrankungen sowie Wirkstoffe zur Beeinflussung von Vorgängen im Unterhautfettgewebe. Zu transdermal applizierbaren Wirkstoffen gehören z.B. Analgetika, Antiarrhythmika, Narkotika und deren Antagonisten, Neuroleptika, Hormone bzw. Hormonersatzstoffe, Antidepressiva, Tranquillantien, Hypnotika, Psychostimulantien, Antiparkinsonmittel, Ganglienblocker, Sympatomimetika, Alpha-Sympatholytika, Beta-Sympatholytika, Antisympathotonika, Antiasthmatika, Antiemetika, Appetitzügler, Diuretika oder Wirkstoffe zur Gewichtsreduzierung etc.. Bedingt durch die erfindungsgemäße geringe Schichtdicke des Systems gelten als bevorzugte Wirkstoffe diejenigen, die schon bei sehr geringer Konzentration ihre Wirkung zeigen.

Beispiele für diese bevorzugten Wirkstoffe sind Steroide wie Estradiol, Estriol, Progesteron, Norethisteron, Norethindron, Levonorgestrel und ihre Derivate sowie Ethynodioldiacetat, Norgestamat, Gestadene, Desogestrel, Demegestron, Promegestron, Testosteron, Hydrocortisone und deren Derivate; Nitro- Verbindungen wie Amylnitrat, Nitroglycerin, Isosorbiddinitrat; Amin- Verbindungen wie Nikotin, Chlorpheniramin, Terfenadin und Triprolidin; Oxicam-Derivate wie Piroxicam; Mucopolysaccharidasen wie Thiomucase; Opioide wie Buprenorphin, Morphin, Fentanyl sowie deren Salze, Derivate oder Analoge, Naloxon, Kodein, Dihydroergotamin, Lysergsäurederivate, Pizotilin, Salbutamol, Terbutalin; Prostaglandine wie die der PGA, PGB, PGE und PGF-Serie, z.B. Misoprostol und Enprostil, Omoeprazol, Imipramin; Benzamide wie Metoclopramine und Scopolamin; Peptide und Wachstumsfaktoren wie EGF, TGF, PDGF etc.; Somatostatin; Clonidin; Dihydropyridine, wie Nifedipin, Nitrendipin, Verapamil, Diltiazem, Ephedrin, Propanolol, Metoprolol, Sprironolacton; Thiazide wie Hydrochlorothiazid und Flunarizin.
Zur Wundbehandlung werden blutstillende Wirkstoffe und wundreinigende Stoffe, wie beispielsweise Enzyme, Antiseptika, Desinfizientia und Antibiotika, schmerzstillende Mittel und anästhesierende Wirkstoffe sowie wundheilungsfördernde Wirkstoffe zur Anregung der Granulation, zur Induzierung der Gefäßbildung oder zur Förderung der Epithelisierung eingesetzt.

In einer bevorzugten Ausführungsform zur transdermalen Anwendung enthält die Filmschicht ein Steroidhormon, vorzugsweise Estradiol allein oder in Kombination mit anderen Arzneistoffen, das bei transdermaler Applikation zur Hormonsubstitution in der Postmenopause oder zur Behandlung der Osteoporose eingesetzt wird.

Andererseits kann die Vorrichtung zur Abgabe von Estradiol auch auf chronischen Wunden, beispielsweise Ulcera Cruris, zur Wundbehandlung eingesetzt werden.

In einer weiteren bevorzugten Ausführungsform der erfindungsgemäßen Vorrichtung enthält die Filmschicht pflanzliche Zubereitungen wie beispielsweise Extrakte oder Tinkturen. Diese können verwendet werden zur Behandlung lokaler Hauterkrankungen, wie beispielsweise Eichenrindenextrakt, Walnußextrakt, Arnikablütentinktur, Hamamelisrindenextrakt, Spitzwegerichextrakt, Stiefmütterchenextrakt, Thymian- oder Salbeiextrakt, zur Behandlung von geschädigter und verletzter Haut, wie beispielsweise Johanniskrauttinktur, Sonnenhutkrauttinktur, Kamillenblütenextrakt oder Ringelblumenblütentinktur, sowie zur Pflege von strapazierter und geschädigter Haut, wie beispielsweise Birkenblätterextrakt, Brennesselextrakt, Huflattichextrakt, Beinwelltinktur, Schachtelhalmextrakt oder Aloe Vera Extrakt.
Pflanzliche Zubereitungen können aus der Filmschicht aber auch abgegeben werden zur intradermalen Behandlung von Erkrankungen, wie beispielsweise Roßkastanien- und Mäusedornextrakte bei Venenleiden , oder Arnika-, Ringelblumen- und Capsicumextrakte und -tinkturen bei Prellungen, Verstauchungen oder Blutergüssen. Pflanzliche Zubereitungen im erfindungsgemäßen System können aber auch in der transdermalen Therapie eingesetzt werden, so beispielsweise Ginsengextrakt bei Altersbeschwerden, Baldriantinktur, Melissen- und Hopfenextrakt zur Beruhigung bei Überreizung, Schlafstörungen und Streß, Cola- und Teeextrakte zur Erzielung einer anregenden Wirkung oder Weißdornextrakt zur Kreislaufstabilisierung.

In besonderen Fällen, in denen der Wirkstoff bzw. der Wirkstoffextrakt selbst filmbildende Eigenschaften besitzt, besteht die Filmschicht allein aus dem Wirkstoff bzw. dem entsprechenden Extrakt.
In einer bevorzugten Ausführungsform besteht die Filmschicht des erfindungsgemäßen Systems aus Tabakpulverextrakt. Eine derartige Vorrichtung kann von Rauchern verwendet werden als Alternative zu Tabak, Zigaretten und ähnlichen Rauchwaren. Dieses System ist auch für den Einsatz einer hier nicht erschöpfend aufgezählten Anzahl filmbildender pflanzlicher und tierischer Extrakte geeignet.

Eine weitere besondere Ausführungsform bezieht sich auf den Einsatz des erfindungsgemäßen Systems als Träger von Narkotika, Psychopharmaka und Mittel zur Behandlung von Alzheimer und seniler Demenz. Diese hochpotenten Arzneistoffe verlangen ein System mit gewährleisteten Trageigenschaft über mehrere Tage und eine abfallfreie Herstellung. Diesen Forderungen werden durch das Siebdruckverfahren der wirkstoffhaltigen Filmschicht wie auch durch Flexibilität und Schichtdicke des Systems Rechnung getragen.

Erfindungsgemäß sind die einzelnen Filmschichtsegmente ebenfalls mit Hilfe des Siebdruckverfahrens in Form von Farben, Buchstaben, Zahlen, Datum, Codes, Piktogrammen etc. kennzeichenbar.
Daneben ergibt sich zwangsläufig die Möglichkeit der Einfärbung der Filmschicht durch lösliche Farbstoffe bzw. Pigmente. Des weiteren ist auch die Möglichkeit eines vollkommen transparenten Systems gegeben.

Die Herstellung des erfindungsgemäßen Systems kann wie nachstehend beschrieben erfolgen:
Ein 150 g Papier (100-200 g/m²), hergestellt aus einem von Aluminiumsulfat freien Faserbrei, welcher als Füllstoff außer Stärke Kaolin enthält, wird oberflächlich nit einer Leimlösung bestrichen und getrocknet. Das Bestreichen erfolgt dabei derart, daß nur die Poren der äußersten Papierschicht abgedeckt werden.
Auf diesen so behandelten Papierträger wird mittels Rakel oder Rasterwalze eine Lösung aus Polyvinylalkohol aufgetragen. Anschließend wird getrocknet. Das so erhaltene mit der Trennschicht (Polyvinylalkohol) versehen Papier, welches zur weiteren Verwendung in Form von Rollen vorliegt, dient als Trägerschicht für den jetzt folgenden Druckvorgang, der in Form eines Siebdruckverfahrens vorgenommen wird. Je nach Design des Systems sind unterschiedlich viele Druckschritte und Druckschablonen erforderlich. Das in Figur 2 gezeigte Beispiels hat jedoch keinen einschränkenden Charakter. Mittels Siebdruck wird zuerst mit handelsüblicher Farbe der Name des Systems auf die Trägerschicht gedruckt.
Anschließend erfolgt die Bedruckung der nach der Applikation außen liegenden ersten Schicht der wirkstoffhaltigen Filmschicht. In dem hier dargestellten Beispiel handelt es sich um die Bedruckung mit einer wirkstofffreien Polyacrylatdispersion (Polyacrylate Dispersion 30 per Cent Ph.Eu.) mit 10 % Acetyltriisobutylcitrat, die nach dem Trocknungsvorgang einen 5 µm starken Film bildet. Die Schablone des Siebdruckauftrages ist so dimensioniert, daß durch diesen Druckvorgang flächenförmig das endgültige System definiert wird. Als nächstes erfolgt die Bedruckung der wirkstoffhaltigen Schicht, die direkt flächendeckend auf die Polyacrylatschicht erfolgt. Im folgenden sind Beispiele für die Zusammensetzung dieses Druckmediums aufgeführt (Gewichtsteile):

| | | |
|---|---|---|
| a) | Estriol | 4 |
| | N,N-Diethyl-m-Toluamid | 4 |
| | Aceton | 50 |
| | Eudragit E 30 D | 40 |
| | Plastoid E 35 | 10 |
| | | |
| b) | Buprenorphin | 10 |
| | Isopropyllanolat | 10 |
| | Plastoid E 35 | 65 |
| | | |
| c) | Estradiol | 4 |
| | Polyacrylate Dispersion 30 per Cent (Ph.Eu.) | 100 |
| | Triethylcitrat | 4 |
| | Propylenglycol | 4 |
| | Aceton | 10 |
| | Polyvinylpyrrolidon | 4 |
| | Lecithin | 1 |
| | Ethanol | 20 |

Im Beispiel c) wird zusätzlich eine 5 µm Schicht mit Plastoid E 35 aufgedruckt.

### (Plastoid und Eudragit sind Handelsbezeichnungen der Rh öm GmbH)

Nach dem Bedruckungsvorgang wird die letzte Schicht mit der Schutzschicht versehen.

Einzelheiten, Merkmale und Vorteile der Erfindung werden nachfolgend anhand von Zeichnungen näher erläutert.

Es zeigen:

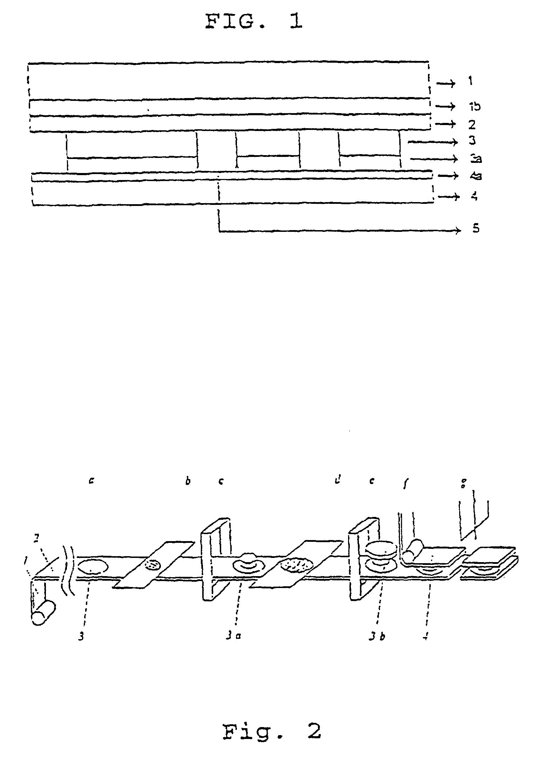

- Figur 1:: eine "Multi-Dose"-Einheit mit drei "Single-Dose"-Systemen im Schnitt;
- Figur 2:: eine schematische Herstellungsvorrichtung;
- Figur 3:: ein transdermales System nach der Erfindung im Schnitt;

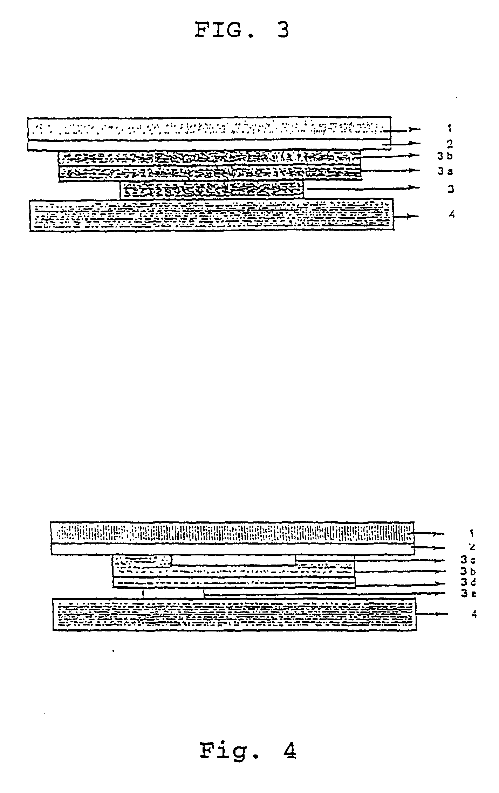

- Figur 4:: ein anderes transdermales Kombinations-System, ebenfalls im Schnitt

In den Figuren bedeuten

| | | |
|---|---|---|
| in Figur 1 | 1 | Trägerschicht |
| | 1b | Sperrschicht |
| | 2 | Trennschicht |
| | 3 | Filmschicht |
| | 3a | Kleberschicht |
| | 4a | Abhäsivschicht |
| | 4 | Schutzschicht |
| | 5 | Perforation |
| in Figur 2 | 1 | Trägerschicht |
| | 2 | Trennschicht |
| | 3 | nicht klebend ausgerüstete dem Substrat abgewandte Schicht des Filmschichtlaminats |
| | 3a | wirkstoffhaltige Schicht des Filmschichtlaminats |
| | 3b | Kleberschicht des Filmschichtlaminats |
| | 4 | Schutzschicht |

auf die durch einen Druckvorgang hergestellte Filmschicht (3) wird bei (a) durch Siebdruck (b) die Filmschicht (3a) auf die Trägerschicht (1) aufgebracht und bei (c) getrocknet. Bei (d) erfolgt ebenfalls durch Siebdruck der Auftrag der Filmschicht (3b), wobei diese in (e) getrocknet wird. Bei (f) wird die Schutzschicht (4) zukaschiert. Bei (g) erfolgt der Schneidvorgang zum fertigen System.

| | | |
|---|---|---|
| In Figur 3 | 1 | Trägerschicht |
| | 2 | Trennschicht |
| | 3a | wirkstofffreie Kleberschicht |
| | 3b | wirkstoffreie Filmschicht |
| | 3 | wirkstoffhaltige Filmschicht |
| | 4 | Schutzschicht |
| | 3a+3b+3 = | Filmschicht Laminat |
| In Figur 4 | 1 | Trägerschicht |
| | 2 | Trennschicht |
| | 3c | bildliche Darstellung |
| | 3d | wirkstoffhaltige Filmschicht Wirkstoff 1 |
| | 3e | wirkstoffhaltige Filmschicht Wirkstoff 2 |
| | 4 | Schutzschicht |
| | 3c+3d+3e= | Filmschicht Laminat |

Selbstverständlich ist die verfahrenstechnisch und zur Erzielung dünnschichtiger und flexibler Systeme besonders nützliche Art der drucktechnischen Erzeugung insbesondere engumrissener wirkstoffhaltiger Schichten nicht auf transdermale therapeutische Systeme beschränkt, die ausgehend von einer Trägerschicht mit einer Trennschicht erzeugt sind, die bei Applikation gelöst bzw. verflüssigt wird, so daß die Trägerschicht entfernt werden kann.

Es ist vielmehr möglich, flächenförmige Systeme zur Abgabe von Wirkstoffen an die Haut durch ein oder mehrere Druckverfahren zu erhalten. Dabei kann der erste Druckvorgang flächenmäßig die Größe des TTS determinieren; alternativ kann aber auch einer der folgenden Druckvorgänge die Größe des Systems determinieren. Der die Größe des Systems bestimmende Druckvorgang kann teilweise oder vollflächig auf einen abhäsiv ausgerüsteten Träger erfolgen.
Zweckmäßigerweise wird zumindest die Rückschicht des fertigen TTS und/oder die mit der Haut in Berührung kommende selbsthaftende Matrixschicht durch das Druckmedium ausgebildet, das im letzteren Fall einen oder mehrere Wirkstoffe aufweisen kann. Die als Druckschicht ausgebildete Rückschicht des TTS kann als Informationsträger zum Bedrucken mit Markierungen oder Kennzeichen ausgenutzt und/oder mit Farbgebung bedruckt werden. Die als Druckschicht ausgebildete Rückschicht des TTS kann mit einer abhäsiv ausgerüsteten Stützfolie versehen sein, deren Abhäsivität geringer ist als die des bedruckten Trägers.
Das heißt, die Erfindung umfaßt Systeme zur kontrollierten Abgabe von Wirkstoffen an die menschliche oder tierische Haut auf der Basis von Systemen, die ausschließlich auf einem oder mehreren Druckverfahren basieren.

Überraschenderweise hat sich gezeigt, daß sich z.B. die Verfahren der Bedruckung von T-Shirts mit Bildern oder Buchstaben insbesondere zwecks Erzielung einer ausreichenden flexiblen Schichtdicke auch zur Herstellung von transdermalen therapeutischen Systemen (TTS) eignen, wenn man die Bedruckung nicht auf Gewebe oder Vlies, sondern auf ein abhäsiv ausgestattetes Substrat vornimmt. Transformierbar auf die Herstellung von kompletten TTS sind auch die Druckverfahren, die zur Herstellung von Abreibebuchstaben wie z.B. Letraset® eingesetzt werden.
Die Dicke des Substrats liegt ähnlich wie bei der mit Trennschicht versehenen Trägerschicht im Bereich von 20-200 µm, vorzugsweise 50-120 µm und in einer besonders bevorzugten Form 60-90 µm, und es ist z.B. durch eine silikonisierte Schicht abhäsiv ausgestattet. Um einen besseren Verbund mit der Haut zu erzielen, kann die dem Substrat anliegende Schicht der Druckmedien klebend ausgestattet sein. Die wirkstoffhaltige Filmschicht kann in einem oder mehreren Einzeldruckschritten aufgetragen werden, wobei ähnlich wie bei einem Mehrfarbendruck auch Teilbereiche des Vordruckes bedruckt werden können. Als Druckverfahren eignen sich wiederum alle dem Fachmann bekannten Druckverfahren, die es gestatten, eine wirkstoffhaltige Filmschicht mit einer geforderten Gewichtskonstanz einheitlich aufzutragen. Die einzelnen Schichten der einzelnen aufzudruckenden Druckschichten können hinsichtich ihres Polymergrundkörpers gleich oder unterschiedlich sein. Es hat sich als vorteilhaft erwiesen, die Filmschicht durch Siebdruck aufzutragen. Hierdurch ist es möglich, auf einem Substrat mehrere in ihrer Größe unterschiedliche, inselartige wirkstoffhaltige Filmschichtbereiche zu erhalten, die sich ähnlich wie bei einem "Mehrfarbendruck" in ihrer Zusammensetzung, Schichtstärke und Wirkstoff (unterschiedliches Pigment) unterscheiden.
Die Dicke der Filmschicht liegt im Bereich von 5-50 µm, vorzugsweise 5-30 µm und in einer besonderen Ausführungsform 10-25 µm, und ist von gummiartiger Konsistenz.

Zwecks Applikation wird das Substrat des Systems entfernt und das verbleibende System auf die Applikationsstelle aufgebracht.
Als Druckmedium kommen die weiter oben genannten filmbildenden Polymeren und Massen in Betracht.

Diese Filmschicht kann in Form eines Laminats durch unten aufgeführte Bestandteile in der der Applikationsstelle zugewandten Schicht haftklebend ausgeführt sein. Als Bestandteile in diesem Sinne eignen sich alle dem Fachmann bekannten handelsüblichen Kleber, die auch zur Wundversorgung in Form von Verbänden und Pflastern eingesetzt werden, wie z.B. Kleber auf der Basis von Acrylaten, Polyisobutylen, Silikonen etc.

Die Bezeichnung "Wirkstoffe" hat wiederum die gleiche Bedeutung wie weiter oben angegeben. Alle genannten Wirkstoffe und Applikationsbesonderheiten gelten wiederum in gleicher Weise wie für das mit Träger- und Trennschicht gebildete System.

Die Herstellung des Systems geschieht analog folgendermaßen:
Ein 150g Papier (100-200g/m²), silikonisiert, dient als Substrat für den Druckvorgang, der in Form des Siebdruckverfahrens vorgenommen wird. Je nach Design des Systems sind unterschiedlich viele Druckschritte und Druckschablonen erforderlich. In der Regel beinhaltet das zuerst eingesetzte Druckmedium den Wirkstoff, wobei dieses Druckmedium selbsthaftend ausgestattet ist. Das danach eingesetzte Druckmedium bildet letztlich die Systemgröße aus. Es ist beispielsweise filmbildend gummiartig. Das gesamte auf dem Substrat aufgetragene Druckbild kann durch eine später als Stützfolie dienende, abhäsiv ausgestattete Polypropylenfolie abgedeckt werden.

## Patentansprüche

1. Schichtsystem zur Applikation einer Wirkstoff abgebenden Filmschicht auf einem Substrat, insbesondere auf der Haut, **gekennzeichnet durch** eine mit einer Trennschicht (2) versehene Trägerschicht (1), eine an die Trennschicht anschließende, den Wirkstoff enthaltende Filmschicht (3) und eine die Filmschicht abdeckende, abhäsiv ausgerüstete Schutzschicht (4), wobei die Trennschicht (2) aus einem Material besteht, dessen Verbund mit der Filmschicht (3) bei Applikation **durch** Konsistenz-Änderung aufgehoben werden kann.

2. Schichtsystem nach Anspruch 1, **dadurch gekennzeichnet, daß** die Trennschicht (2) eine wasserlösliche filmbildende Substanz enthält.

3. Schichtsystem nach Anspruch 1, **dadurch gekennzeichnet, daß** die Trennschicht (2) eine fettlösliche filmbildende Substanz enthält.

4. Schichtsystem nach Anspruch 1, **dadurch gekennzeichnet, daß** die Trennschicht (2) eine sowohl wasser- als auch fettlösliche filmbildende Substanz enthält.

5. Schichtsystem nach Anspruch 1, **dadurch gekennzeichnet, daß** die Trennschicht (2) eine durch Temperaturerhöhung über 40°C verflüssigbare Substanz ist.

6. Schichtsystem nach Anspruch 2-5, **dadurch gekennzeichnet, daß** die Trennschicht (2) eine Schichtdicke von 1-50 µm, vorzugsweise 5-20 µm aufweist.

7. Schichtsystem nach Anspruch 1, **dadurch gekennzeichnet, daß** die Trägerschicht (1) aus einem porösen Material, vorzugsweise Papier besteht.

8. Schichtsystem nach Anspruch 1 oder 7, **dadurch gekennzeichnet, daß** die Trägerschicht (1) eine Schichtdicke von 20- 200 µm, vorzugsweise 100-150 µm aufweist.

9. Schichtsystem nach Anspruch 1, **dadurch gekennzeichnet, daß** die Filmschicht (3) durch einen oder mehrere Bedruckungsvorgänge auf der mit Trennschicht versehenen Trägerschicht (1) gebildet ist.

10. Schichtsystem nach Anspruch 1, **dadurch gekennzeichnet, daß** die wirkstoffhaltige Filmschicht (3) ein Laminat darstellt.

11. Schichtsystem nach Anspruch 10, **dadurch gekennzeichnet, daß** die Einzelschichten in mindestens einem der Parameter Fläche, Schichtdicke und Zusammensetzung unterschiedlich sind und zumindest eine Schicht mindestens einen Wirkstoff beinhaltet.

12. Schichtsystem nach Anspruch 11, **dadurch gekennzeichnet, daß** die Wirkstoffe in der Filmschicht in voneinander. getrennten Bereichen eingebracht sind.

13. Schichtsystem nach Anspruch 1, **dadurch gekennzeichnet, daß** die wirkstoffhaltige Filmschicht (3) schutzschichtseitig selbstklebend ausgestattet ist.

14. Schichtsystem nach Anspruch 1, **dadurch gekennzeichnet, daß** die Filmschicht (3) ein oder mehrere filmbildende Polymere enthält.

15. Schichtsystem nach Anspruch 1, **dadurch gekennzeichnet, daß** die Filmschicht (3) eine Schichtdicke von 5-50 µm, vorzugsweise 5-20 µm besitzt.

16. Schichtsystem nach Anspruch 1, **dadurch gekennzeichnet, daß** die Filmschicht (3) in voneinander beabstandeten Abschnitten auf der Trägerschicht (1) verteilt ist.

17. Verfahren zur Herstellung eines Schichtsystems nach einem der Ansprüche 1 bis 16, **gekennzeichnet durch** einen oder mehrere Druckverfahrensschritte, zur Erzeugung der wirkstoffhaltigen Filmschicht zwischen dem mit Trennschicht versehenen Träger und der abdeckenden Schutzschicht.

18. Verfahren nach Anspruch 17, **dadurch gekennzeichnet, daß** der erste Druckvorgang flächenmäßig die Größe des Schichtsystems determiniert.

19. Verfahren nach Anspruch 17, **dadurch gekennzeichnet, daß** einer der folgenden Druckvorgänge die Größe des Systems determiniert.

20. Verfahren nach Anspruch 17, **dadurch gekennzeichnet, daß** der die Größe des Systems bestimmende Druckvorgang teilweise oder vollständig auf den mit lös- oder verflüssigbarer Trennschicht versehenen Träger erfolgt.

21. Verfahren nach Anspruch 17, **dadurch gekennzeichnet, daß** das Druckmedium die für die Ausbildung einer Applikationsoberfläche der Filmschicht geeignet ist.

22. Verfahren nach Anspruch 17, **dadurch gekennzeichnet, daß** das Druckmedium für die Bildung der mit der Haut in Berührung kommenden selbsthaftenden Matrixschicht geeignet ist.

23. Verfahren nach Anspruch 17, **dadurch gekennzeichnet, daß** das oder ein Druckmedium einen oder mehrere Wirkstoffe beinhaltet.

24. Verfahren nach Anspruch 17, **dadurch gekennzeichnet, daß** die für die Ausbildung der Applikationsoberfläche dienende Druckschicht zwecks Kennzeichnung und/oder Farbgebung bedruckt wird.

## Claims

1. A layered system for application of an active substance-releasing film layer to a substrate, especially to the skin, **characterized by** a substrate (1), which is provided with a separating layer (2), a film layer (3), which is adjacent to the separating layer and which contains the active substance, and a protective layer (4) provided with a non-stick finish and covering the film layer, said separating layer (2) consisting of a material whose bond to the film layer (3) may be abolished, through a change in consistency, upon application.

2. The layered system according to claim 1 **characterized in that** the separating layer (2) comprises a water-soluble film-forming substance.

3. The layered system according to claim 1 **characterized in that** the separating layer (2) comprises a fat-soluble film-forming substance.

4. The layered system according to claim 1 **characterized in that** the separating layer (2) comprises a film-forming substance that is both water-soluble and fat-soluble.

5. The layered system according to claim 1 **characterized in that** the separating layer (2) is a substance that can be liquefied by a temperature elevation to above 40°C.

6. The layered system according to claims 2 to 5 **characterized in that** the separating layer (2) has a layer thickness of 1-50 µm, preferably 5-20 µm.

7. The layered system according to claim 1 **characterized in that** the substrate (1) consists of a porous material, preferably of paper.

8. The layered system according to claim 1 or 7 **characterized in that** the substrate (1) has a layer thickness of 20-200 µm, preferably 100-150 µm.

9. The layered system according to claim 1, **characterized in that** the film layer (3) is formed by one or several printing process steps on the substrate (1), which is provided with a separating layer.

10. The layered system according to claim 1 **characterized in that** the active substance-containing film layer (3) represents a laminate.

11. The layered system according to claim 10 **characterized in that** the individual layers are different with respect to at least one of the parameters area, layer thickness, and composition and that at least one layer comprises at least one active substance.

12. The layered system according to claim 11 '**characterized in that** the active substances in the film layer are introduced in regions separated from each other.

13. The layered system according to claim 1 **characterized in that** the active substance-containing film layer (3) is self-adhesive on the side of the protective layer.

14. The layered system according to claim 1 **characterized in that** the film layer (3) comprises one or several film-forming polymers.

15. The layered system according to claim 1 **characterized in that** the film layer (3) has a layer thickness of 5-50 µm, preferably 5-20 µm.

16. The layered system according to claim 1 **characterized in that** the film layer (3) is distributed in spaced apart sections on the substrate (1).

17. A process for the production of a layered system according to any one of claims 1 to 16, **characterized by** one or more printing process steps, for producing the active substance-containing film layer between the substrate, which substrate has been provided with a separating layer, and the covering protective layer.

18. The process according to claim 17 **characterized in that** the first printing procedure determines the size of the layered system with respect to its area.

19. The process according to claim 17 **characterized in that** the size of the system is determined by one of the subsequent printing procedures.

20. The process according to claim 17 **characterized in that** the printing process which determines the size of the system is effected partially or completely onto the substrate which has been provided with a soluble or liquefiable separating layer.

21. The process according to claim 17 **characterized in that** the printing medium is suitable for the formation of an application surface of the film layer.

22. The process according to claim 17 **characterized in that** the printing medium is suitable for the formation of the self-adhesive matrix layer which comes into contact with the skin.

23. The process according to claim 17 **characterized in that** the, or a, printing medium contains one or more active substances.

24. The process according to claim 17 **characterized in that** the print layer which serves to form the application surface is printed for the purpose of marking or designating and/or colouring.

## Revendications

1. Système multicouche pour l'application d'une couche sous forme de film libérant une substance active sur un substrat, en particulier sur la peau, **caractérisé par** une couche de support (1) munie d'une couche de séparation (2), une couche sous forme de film (3) se raccordant à la couche de séparation et contenant la substance active et une couche de protection (4) rendue adhésive, qui recouvre la couche sous forme de film, dans lequel la couche de séparation (2) est constituée d'une matière dont la liaison avec la couche sous forme de film (3) lors de l'application peut être supprimée par modification de la consistance.

2. Système multicouche selon la revendication 1, **caractérisé en ce que** la couche de séparation (2) contient une substance filmogène hydrosoluble.

3. Système multicouche selon la revendication 1, **caractérisé en ce que** la couche de séparation (2) contient une substance filmogène liposoluble.

4. Système multicouche selon la revendication 1, **caractérisé en ce que** la couche de séparation (2) contient une substance filmogène aussi bien hydrosoluble que liposoluble.

5. Système multicouche selon la revendication 1, **caractérisé en ce que** la couche de séparation (2) est une substance liquéfiable sous l'influence d'une élévation de la température au-delà de 40 °C.

6. Système multicouche selon les revendications 2 à 5, **caractérisé en ce que** la couche de séparation (2) présente une épaisseur de couche de 1 à 50 µm, de préférence de 5 à 20 µm.

7. Système multicouche selon la revendication 1, **caractérisé en ce que** la couche de support (1) est constituée d'une matière poreuse, de préférence de papier.

8. Système multicouche selon la revendication 1 ou 7, **caractérisé en ce que** la couche de support (1) présente une épaisseur de couche de 20 à 200 µm, de préférence de 100 à 150 µm.

9. Système multicouche selon la revendication 1, **caractérisé en ce que** la couche sous forme de film (3) est formée par un ou plusieurs processus d'impression sur la couche de support (1) munie de la couche de séparation.

10. Système multicouche selon la revendication 1, **caractérisé en ce que** la couche sous forme de film (3) contenant la substance active représente un stratifié.

11. Système multicouche selon la revendication 10, **caractérisé en ce que** les couches individuelles sont différentes en ce qui concerne au moins un des paramètres que sont la surface, l'épaisseur de couche et la composition, et au moins une couche renferme au moins une substance active.

12. Système multicouche selon la revendication 11, **caractérisé en ce que** les substances actives dans la couche sous forme de film sont incorporées dans des zones séparées les unes des autres.

13. Système multicouche selon la revendication 1, **caractérisé en ce que** la couche sous forme de film (3) contenant la substance active est rendue auto-adhésive du côté tourné vers la couche de protection.

14. Système multicouche selon la revendication 1, **caractérisé en ce que** la couche sous forme de film (3) contient un ou plusieurs polymères filmogènes.

15. Système multicouche selon la revendication 1, **caractérisé en ce que** la couche sous forme de film (3) possède une épaisseur de couche de 5 à 50 µm, de préférence de 5 à 20 µm.

16. Système multicouche selon la revendication 1, **caractérisé en ce que** la couche sous forme de film (3) est subdivisée en sections disposées les unes à l'écart des autres sur la couche de support (1).

17. Procédé pour la fabrication d'un système multicouche selon l'une quelconque des revendications 1 à 16, **caractérisé par** une ou plusieurs étapes opératoires d'impression pour l'obtention de la couche sous forme de film contenant la substance active entre le support muni de la couche de séparation et la couche protectrice de recouvrement.

18. Procédé selon la revendication 17, **caractérisé en ce que** le premier processus d'impression détermine, en ce qui concerne la surface, la dimension du système multicouche.

19. Procédé selon la revendication 17, **caractérisé en ce qu'**un des processus d'impression suivants détermine la dimension du système.

20. Procédé selon la revendication 17, **caractérisé en ce que** le processus d'impression déterminant la dimension du système a lieu en partie ou complètement sur le support muni de la couche de séparation soluble ou liquéfiable.

21. Procédé selon la revendication 17, **caractérisé en ce que** le milieu d'impression est approprié pour la réalisation d'une surface d'application de la couche sous forme de film.

22. Procédé selon la revendication 17, **caractérisé en ce que** le milieu d'impression est approprié pour la formation de la couche matricielle auto-adhésive entrant en contact avec la peau.

23. Procédé selon la revendication 17, **caractérisé en ce que** le ou un milieu d'impression renferme une ou plusieurs substances actives.

24. Procédé selon la revendication 17, **caractérisé en ce que** la couche d'impression utilisée pour la réalisation de la surface d'application est imprimée à des fins de caractérisation et/ou de coloration.
